# EUROPEAN PATENT SPECIFICATION

(11) **EP 2 051 643 B1**
(45) Date of publication and mention of the grant of the patent: **09.11.2011**
(21) Application number: 07787976.5
(22) Date of filing: 27.07.2007
(51) Int. Cl.: A61B 17/32

(54) **CUTTING INSTRUMENTS FOR ULTRASONIC BONE SURGERY**
SCHNEIDINSTRUMENTE FÜR ULTRASCHALL-KNOCHENCHIRURGIE
INSTRUMENTS DE DÉCOUPE À DES FINS DE CHIRURGIE OSSEUSE PAR ULTRASONS

(30) Priority: 27.07.2006 IT TO20060113 U
(43) Date of publication of application: 29.04.2009
(73) Proprietor: Blus, Cornelio, 14030 Montemagno (Asti) (IT)
(72) Inventor: Blus, Cornelio, 14030 Montemagno (Asti) (IT)
(74) Representative: Fioravanti, Corrado
(86) International application number: PCT/EP2007/057761
(87) International publication number: WO 2008/012359

(56) References cited:
- EP-A- 0 456 470
- EP-A- 1 110 509
- EP-A- 1 138 264
- WO-A-2006/052903
- WO-A-2006/059120
- US-A1- 2006 030 797

## Description

The present invention relates to cutting instruments for use in ultrasonic bone surgery. The instruments according to the invention are intended for use, particularly but not exclusively, in the fields of orthopaedic surgery, neurosurgery, otorhinolaringologic surgery, maxillofacial surgery, dental surgery and veterinary surgery.

The ultrasonic technique is widespread in bone surgery, where it is generally used also because it does not cause undesired cuts in the flesh. There are known surgical devices containing electrically supplied piezoelectric transducers which cause vibration of a metal instrument that can cut the bone or separate biological tissues. See, for example, patent publications US-2 984 241, US-4 188 952 and EP-0 238 667.

WO-A2-2006/052903 discloses a surgical instrument having the features defined in the preamble of claim 1.

It is an object of the invention to provide a cutting instrument capable of reaching more easily inner zones of the human body which are difficult to access, such as narrow and deep recesses. Another object of the invention is to provide a cutting instrument allowing the surgeon to have a clear vision of both the cutting end of the instrument and the whole operating zone.

These objects are achieved, in accordance with the present invention, by a surgical instrument having the features defined in claim 1. Preferred embodiments of the invention are defined in the dependent claims.

The advantages of the invention will become apparent from the following detailed specification, given by way of example, reference being made to the accompanying drawings, in which:
figure. 1 is side view of a first embodiment of a surgical cutting instrument according to a preferred embodiment of the present invention;
figure 2 is a partial view of the free end of the instrument of figure 1 looking in the direction of arrow II in figure 1;
figure 3 is a partial view similar to that of figure 2, of a second embodiment of an instrument according to the invention;
figure 4 is a side view of a third embodiment of a surgical instrument according to the invention;
figures 5 and 6 are partial views similar to those of figures 2 and 3, of a fourth and a fifth embodiment of the surgical instrument;
figures 7 and 8 are side views of a sixth and a seventh embodiment of the surgical instrument according to the invention;
figure 9 is a perspective view schematically showing an apparatus for vibrating the instruments of figures 1 to 9 at ultrasonic frequencies, and
figure 10 schematically shows a control panel of the apparatus of figure 9.

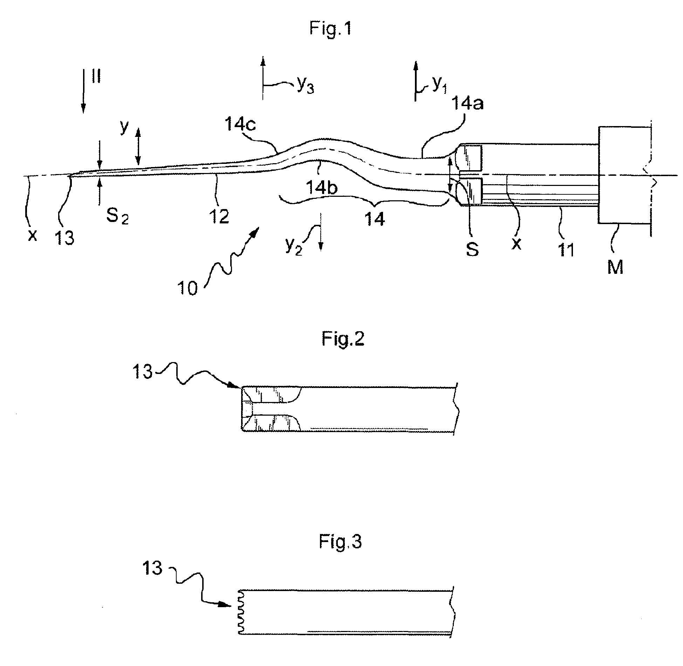

A first embodiment of a surgical instrument for ultrasonic bone surgery according to the invention, shown in figures 1 and 2, is indicated as a whole at 10. The instrument 10, consisting of an elongate body made of titanium alloy, preferably titanium grade 5 alloy, extends along a central axis x having a succession of straight and curved lengths lying in a plane herein defined "vertical". The instrument 10 comprises a proximal length or locking tang 11 of rectilinear shape, a thin, straight distal length 12 with a sharp cutting end 13 and an intermediate length 14 which includes three successive curved portions with respective alternating concavities, as described in detail hereinafter.

The tang 11, substantially cylindrical and having an increased diameter with respect to that of the distal length 12, is adapted to be clamped or screwed or otherwise steadily mounted onto a cylindrical handpiece M (shown only in part) that the surgeon will grip. The handpiece is part of a medical ultrasonic apparatus which electrically supplies a set of piezoelectric transducers housed in the handpiece, so as to vibrate the instrument 10 at a frequency in the ultrasonic range. The constructional and functional features of the aforementioned apparatus are not *per se* relevant for the understanding of the invention and will not therefore be described in detail. Suffice it here to say that said apparatus allows to adjust the input power and the frequency of the vibrations imparted to the instrument.

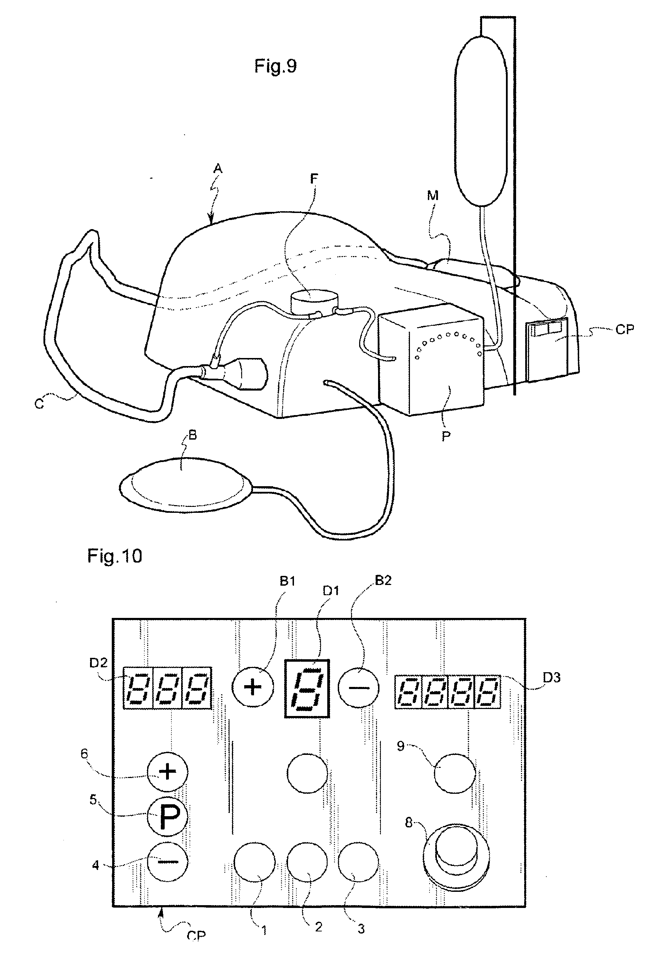

Figure 9 shows by way of example an ultrasonic apparatus A with a handpiece M connected through a cable C. Indicated F is a flowmeter for measuring the flow rate of a cooling physiological solution supplied by means of a peristaltic pump P. Apparatus A is equipped with an on/off foot pedal B and a control panel CP (figure 10) comprised of keys 1, 2, 3 for selecting frequency channels as a function of the type of instrument being used, keys 4, 5, 6 for adjusting the flow rate supplied by the pump P, a knob 8 for selecting the frequency of the instrument mounted on the handpiece. By acting on the knob 8, one varies the frequency of the electric power supply, with a consequent impedance variation that causes a change in the frequency of vibration of the instrument. Keys B 1 and B2 allow to select the power level supplied to the handpiece M, whereas displays D1 and D2 show the selected power and flow rate levels. A further display D3 shows the time the apparatus has been used. It can be reset by a key 9.

Vibrations are transmitted to the tang 11 in form of pulses along the axis x and are propagated and amplified through the intermediate length 14 and transmitted to the distal length 12 and the cutting end 13 that is brought in contact with the biological tissue to cut.

The distal length 12 extends in a direction substantially parallel to or coincident with that of the tang 11. As shown, while the tang 11 has a substantially uniform thickness, the intermediate and distal lengths 14 and 12 are progressively flattened and get thinner when measured in directions y1, y2, y3 which are perpendicular, in each point, to the central axis x and lie in the aforesaid vertical plane.

The intermediate length 14 has a first curved portion 14a with its concavity directed upwardly in the vertical plane, a second curved portion 14b with a downwardly facing concavity and a third curved portion 14c with an upwardly directed concavity radiused to the distal length 12 so that the latter is substantially aligned with or parallel to the tang 11. The bending radius of the third curved portion 14c is comparable to that of the first curved portion 14a, but definitely greater than that of the second curved portion 14b.

Preferably the thickness S, as measured in direction y at the junction between the tang 11 and the intermediate length 14, ranges between 2.0 and 3.0 mm, whereas the thickness S2 of distal length 12, near the sharp cutting end 13 is preferably ranging between 0.4 and 0.6 mm. The overall length of the instrument, without the tang, is preferably of about 30 mm.

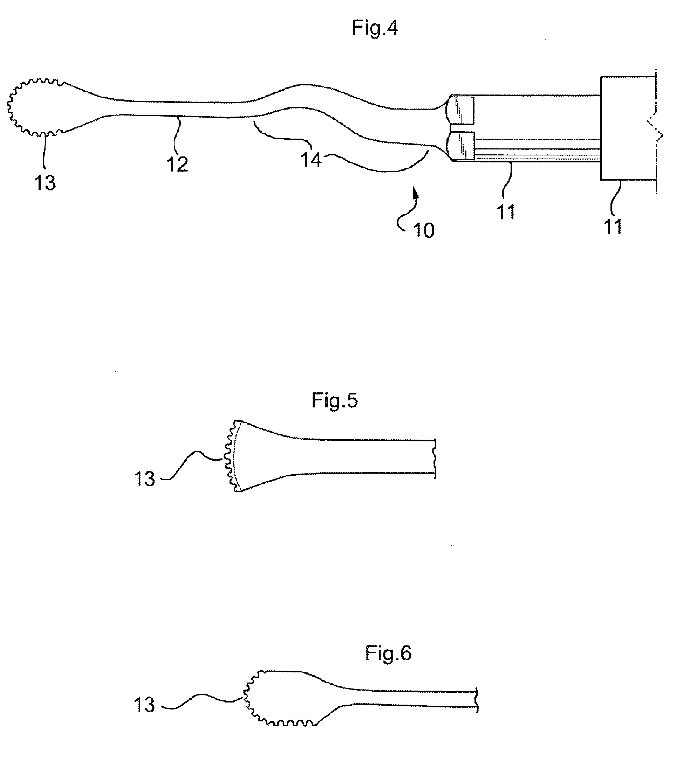

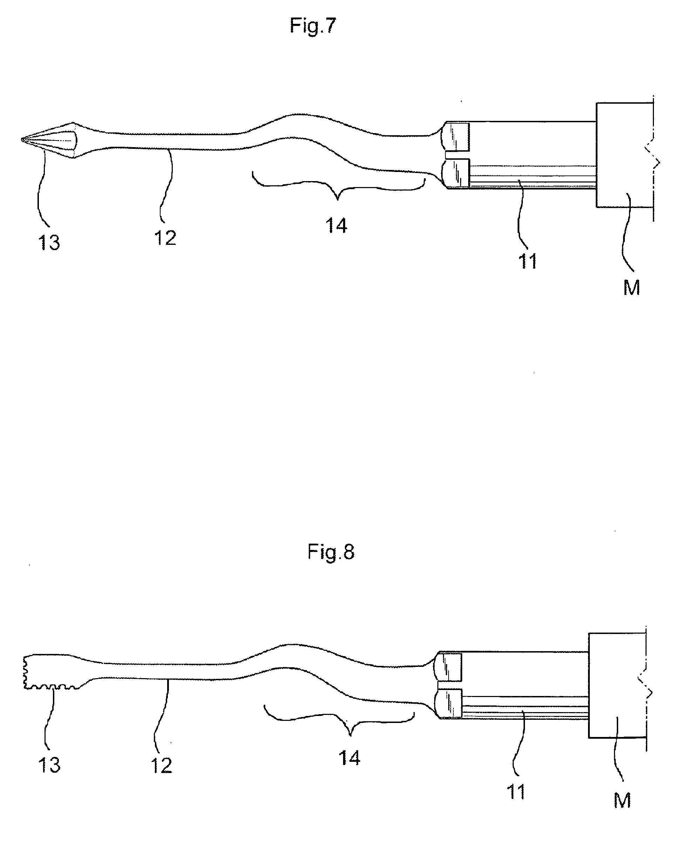

The instruments shown in figures 3 to 8 differ from those of figures 1 and 2 in the particular shape of the sharp cutting end 13, which may take different shapes according to the special purpose of the instrument (for example saw-toothed, chisel-like, triangular, with a perforating tip, etc.).

Tests carried out so far by the Applicant have proven that, owing to the shape of the instrument as described herein above, the instrument allows to cut bone located in sites otherwise difficult to reach, while letting the surgeon clearly observe the position and motion of the cutting end. More particularly, instruments made of titanium alloy have displayed surprising performances if used with ultrasonic apparatus of the above mentioned type within a frequency range between about 22 kHz and about 36kHz. Best results have been attained using on the apparatus an input power ranging between 20 and 90 W, particularly between 42 and 90 W, with a sinusoidal wave. It has been noted that the cut resulting from the use of an instrument of the invention is particularly accurate and thin.

The above results have been attained with instruments manufactured by chip-forming machining from a blank. The same instruments are then subjected to a normalizing step in order to preserve the molecular properties of the titanium alloy. It is believed that this machining and this treatment confer better elastic properties to the instrument in terms of wider oscillations of its free cutting end.

The invention is not intended to be limited to the embodiments described and illustrated herein, which should be considered as examples of a surgical cutting instrument; rather, the invention may be modified with regard to constructional and functional details, particularly concerning the shape of the sharp cutting end.

## Claims

1. A surgical instrument (10) for cutting bone by vibrating at ultrasonic frequencies, including an elongate metal body extending along a central axis (x), the body lying in a plane and having a succession of straight and curved lengths, the instrument being made of titanium alloy and comprising:
- a straight tang (11) adapted to be locked to a surgical device (M) capable of imparting to the instrument vibrations at frequencies ranging between about 22 kHz and about 36 kHz,
- a straight distal length (12) substantially aligned with or parallel to the tang (11) but thinner than the tang and having a sharp free end (13),
- an intermediate length (14) between the tang (11) and the distal length (12), **characterised in that** the intermediate length has three successive curved portions (14a, 14b, 14c) which include:
a first curved portion (14a) having its concavity directed in a first direction (y1) substantially perpendicular to the central axis (x),
a second curved portion (14b) having its concavity directed in a second direction (y2) which is opposite to the first direction (y1) and perpendicular to the central axis (x), and
a third curved portion (14c) having its concavity directed in a third direction (y3) which is opposite to the second direction (y2) and perpendicular to the central axis (x),
and wherein the intermediate (14) and distal (12) lengths are progressively flattened and get thinner when measured in said directions (y1, y2, y3).

2. A surgical instrument according to claim 1, **characterised in that** the instrument (10) is made of titanium grade 5 alloy.

3. A surgical instrument according to claim 1 or 2, **characterised in that**
the thickness (S) of the instrument, measured in said perpendicular direction (y) at the junction between the tang (11) and the intermediate length (14) ranges between about 2.0 and about 3.0 mm, and that
the thickness (S2) of the distal length (12) near the sharp free end (13) ranges between about 0.4 and about 0.6 mm.

4. A surgical instrument according to any one of the preceding claims, **characterised in that** the overall length of the instrument (10), without the tang (11), is about 30 mm.

## Patentansprüche

1. Chirurgisches Instrument (10) zum Schneiden von Knochen durch Vibrieren mit Ultraschallfrequenzen, umfassend einen länglichen Metallkörper, der sich entlang einer Mittelachse (x) erstreckt, wobei der Körper in einer Ebene liegt und eine Abfolge von geraden und gekrümmten Längen aufweist, wobei das Instrument aus einer Titanlegierung gefertigt ist und umfasst:
- einen geraden Stiel (11), der angepasst ist, mit einer chirurgischen Vorrichtung (M) verriegelt zu werden, welche in der Lage ist, an das Instrument Vibrationen zu übermitteln mit Frequenzen, die zwischen etwa 22 kHz und etwa 36 kHz liegen,
- eine gerade distale Länge (12), die im Wesentlichen mit dem Stiel (11) fluchtet oder parallel dazu ist, jedoch dünner als der Stiel ist und ein scharfes freies Ende (13) aufweist,
- eine Zwischenlänge (14) zwischen dem Stiel (11) und der distalen Länge (12),
**dadurch gekennzeichnet, dass** die Zwischenlänge drei aufeinander folgende gekrümmte Abschnitte (14a, 14b, 14c) aufweist, welche umfassen:
- einen ersten gekrümmten Abschnitt (14a), dessen Wölbung in eine erste Richtung (y1) weist, die im Wesentlichen orthogonal zu der Mittelachse (x) ist,
- einen zweiten gekrümmten Abschnitt (14b), dessen Wölbung in eine zweite Richtung (y2) weist, die der ersten Richtung (y1) entgegengesetzt und orthogonal zu der Mittelachse (x) ist, und
- einen dritten gekrümmten Abschnitt (14c), dessen Wölbung in eine dritte Richtung (y3) weist, die der zweiten Richtung (y2) entgegengesetzt und orthogonal zu der Mittelachse (x) ist,
und wobei die Zwischenlänge (14) und die distale Länge (12), gemessen in die Richtungen (y1, y2, y3), fortschreitend abflachen und dünner werden.

2. Chirurgisches Instrument nach Anspruch 1, **dadurch gekennzeichnet, dass** das Instrument (10) aus Titan Grad 5 Legierung gefertigt ist.

3. Chirurgisches Instrument nach Anspruch 1 oder 2, **dadurch gekennzeichnet, dass** die Dicke (S) des Instruments, gemessen in die orthogonale Richtung (y), an der Verbindung zwischen dem Stiel (11) und der Zwischenlänge (14) zwischen etwa 2,0 und etwa 3,0 mm liegt, und dass die Dicke (S2) der distalen Länge (12) nahe dem scharfen freien Ende (13) zwischen etwa 0,4 und etwa 0,6 mm liegt.

4. Chirurgisches Instrument nach einem der vorhergehenden Ansprüche, **dadurch gekennzeichnet, dass** die Gesamtlänge des Instruments (10), ohne den Stiel (11), etwa 30 mm beträgt.

## Revendications

1. Instrument chirurgical (10) servant à découper un os par vibration à des fréquences ultrasonores, comprenant un corps métallique allongé s'étendant le long d'un axe central (x), le corps reposant dans un plan et présentant une succession de longueurs linéaires et incurvées, l'instrument étant fabriqué à partir d'alliage de titane et comprenant :
- une languette droite (11) conçue pour être bloquée sur un dispositif chirurgical (M) capable d'imprimer à l'instrument des vibrations à des fréquences comprises entre environ 22 kHz et environ 36 kHz,
- une longueur distale linéaire (12) sensiblement alignée sur la languette (11) ou parallèle à celle-ci, mais plus mince que la languette et présentant une extrémité libre pointue (13),
- une longueur intermédiaire (14) entre la languette (11) et la longueur distale (12), **caractérisée en ce que** la longueur intermédiaire présente trois parties incurvées successives (14a, 14b, 14c) qui comprennent :
une première partie incurvée (14a) dont la concavité est dirigée dans une première direction (y1) sensiblement perpendiculaire à l'axe central (x),
une deuxième partie incurvée (14b) dont la concavité est dirigée dans une deuxième direction (y2) qui est opposée à la première direction (y1) et perpendiculaire à l'axe central (x), et
une troisième partie incurvée (14c) dont la concavité est dirigée dans une troisième direction (y3) qui est opposée à la deuxième direction (y2) et perpendiculaire à l'axe central (x),
et dans lequel les longueurs intermédiaire (14) et distale (12) sont progressivement aplaties et s'amincissent lorsqu'elles sont mesurées dans lesdites directions (y1, y2, y3).

2. Instrument chirurgical selon la revendication 1, **caractérisé en ce que** l'instrument (10) est réalisé à partir d'alliage de titane de grade 5.

3. Instrument chirurgical selon la revendication 1 ou 2, **caractérisé en ce que**
l'épaisseur (S) de l'instrument, mesurée dans ladite direction perpendiculaire (y) au niveau de la jonction entre la languette (11) et la longueur intermédiaire (14) est comprise entre environ 2,0 et environ 3,0 mm, et que
l'épaisseur (S2) de la longueur distale (12) près de l'extrémité libre pointue (13) est comprise entre environ 0,4 et environ 0,6 mm.

4. Instrument chirurgical selon l'une quelconque des revendications précédentes, **caractérisé en ce que** la longueur hors tout de l'instrument (10), sans la languette (11), est d'environ 30 mm.
